# NOUVEAU FASCICULE DE BREVET EUROPEEN

(11) **EP 0 733 614 B2**
(45) Date de publication et mention de la décision concernant l'opposition: **13.01.2010**
(45) Mention de la délivrance du brevet: 23.05.2001
(21) Numéro de dépôt: 96400625.8
(22) Date de dépôt: 22.03.1996
(51) Int. Cl.: C07C 29/62, C07C 29/38

(54) **Réactif et procédé utiles pour greffer un groupement difluorométhyle substitué sur un composé comportant au moins une fonction électrophile**
Reagenz und Verfahren zum Anbringen einer substituierten Difluoromethylgruppe an einer Verbindung mit mindenstens einer elektrophilen Funktion
Reagent and process useful for attaching a substituted difluoromethyl group onto a compound with at least one electrophilic function

(30) Priorité: 24.03.1995 FR 9503512; 29.12.1995 FR 9515763
(43) Date de publication de la demande: 25.09.1996
(73) Titulaire: RHODIA CHIMIE, 92408 Courbevoie Cédex (FR)
(72) Inventeur: Forat, Gérard, 69003 Lyon (FR); Mas, Jean-Manuel, 69390 Millery (FR); Saint-Jalmes Laurent, 69330 Meyzieu (FR)
(74) Mandataire: Bernasconi, Jean Raymond

(56) Documents cités:
- EP-A- 0 307 519
- US-A- 4 808 748
- US-A- 4 990 699
- JOURNAL OF FLUORINE CHEMISTRY, vol. 45, no. 3, Décembre 1989, LAUSANNE, CH, pages 431-433, XP002007099 G.P STAHLY: "Trifuoromethylation of 1,3,5-trinitrobenzene"
- JOURNAL OF THE CHEMICAL SOCIETY, PERKIN TRANSACTIONS 1, no. 7, Juillet 1990, LETCHWORTH, GB, pages 1951-1957, XP002007100 M. TORDEUX, ET AL.: "Reactions of trifluoromethyl bromide and related halides: part 9. Comparison between additions to carbonyl compounds, enamines, and sulphur dioxide in the presence of zinc"
- G. A. WHEATON, D.J. BURTON: 'Generation and Reactions of Halodifluoromethide Ions' J. ORG. CHEM. vol. 43, no. 13, 1978, pages 2643 - 2651

## Description

La présente invention concerne un réactif et un procédé pour greffer un groupement difluorométhyle substitué sur un composé comportant au moins une fonction électrophile. Elle concerne plus particulièrement une technique pour perfluoroalcoyler différents composés par des réactions de substitution nucléophile ou d'addition typiquement réalisées par des dérivés organométalliques.

Les techniques de perfluoroalcoylation, ou les techniques équivalentes, utilisent en général des dérivés du type iodure de perfluoroalcoyle, en présence de zinc. Cette technique est donc coûteuse, tout en nécessitant des installations de traitement des rejets métalliques qu'il convient de traiter car le zinc est un polluant important des cours d'eau.

Les autres techniques, où le radical perfluoroalcoyle ne forme pas un intermédiaire réactif stabilisé du type organométallique, sont en général difficiles à mettre en oeuvre en raison de la très faible stabilité des anions perfluorés libres dans les milieux réactionnels. Ces derniers conduisent en général à des produits du type carbène, par perte d'un de leurs substituants.

C'est pourquoi un des buts de la présente invention est de fournir un réactif qui permette une perfluoroalcoylation selon un mécanisme du type faisant intervenir un carbanion, sans faire appel à des organométalliques de métaux de transition comme le zinc.

On a souvent cherché à utiliser comme source de radicaux perfluoroalcoyle, plus généralement de radicaux trifluorométhyle, des acides perfluorocarboxyliques, en mettant en oeuvre des réactions de décomposition visant à éliminer le fragment carboxylique desdits acides en libérant du bioxyde de carbone. Toutefois, les succès qui avaient été obtenus étaient très mitigés et utilisaient des systèmes catalytiques particulièrement compliqués. Les radicaux perfluoroalcoyle ou leurs équivalents engendrés par la décomposition desdits acides perfluorocarboxyliques étaient en outre instables dans le milieu réactionnel et nécessitaient l'emploi d'agents stabilisants.

G. Stahly a également rapporté dans Journal of Fluorine Chemistry, 45 (1989), 431-433 et dans US-A-4 990 699 que la décomposition thermique de sels d'acides perfluoroalcanoïques en présence de composés aromatiques tels que le 1,3,5-trinitrobenzène conduit à la formation d'anions trifluorométhyle CF₃⁻ mis en évidence par la formation d'un complexe de Meisenheimer. Le complexe peut être converti ultérieurement par oxydation pour donner le dérivé perfluroalkylé sur le noyau aromatique correspondant.

Toutefois, la nécessité de procéder à cette oxydation rend cette voie de perfluoroalkylation de dérivés aromatiques fastidieuse.

US 4 808 748 décrit un procédé de trifluorométhylation de composés aromatiques par réaction du bromure ou de l'iodure dudit composé aromatique avec du trifluoroacétate de potassium en présence d'ions cuivreux, de préférence en conditions anhydres.

La présente invention se propose d'obvier aux inconvénients des procédés existants en fournissant un réactif non nocif pour l'environnement et capable de conduire aux produits désirés avec un rendement satisfaisant.

Au cours de l'étude qui a mené à la présente invention, il a été démontré qu'une réaction de fluoroalcoylation était possible avec un sel d'acide fluorocarboxylique, sans catalyseur et sans agent susceptible de stabiliser les divers intermédiaires envisagés obtenus lors de la décomposition des différents acides perfluorocarboxyiiques.

Il est apparu que, pour obtenir ainsi une décomposition des acides fluorocarboxyliques, deux conditions étaient essentielles ; l'une est le choix du solvant, et l'autre la teneur en impuretés du mélange constituant le réactif selon la présente invention. Ainsi, il a pu être démontré le rôle absolument critique de la teneur en hydrogènes labiles du système, ou plus exactement en protons libérables, qui doit être inférieure à la teneur en groupes fluorés libérés par la décomposition des sels d'acides fluorocarboxyliques.

Par hydrogène labile et proton libérable, on entend un atome d'hydrogène susceptible d'être arraché par une base forte sous forme de proton. En pratique, il s'agit des protons des fonctions acides qui présentent un pKa inférieur à environ 20 (par "environ", on souligne que le nombre 20 ne présente qu'un chiffre significatif).

Les buts précités et d'autres, qui apparaîtront par la suite, sont atteints au moyen d'un réactif nucléophile utile pour greffer un groupement difluorométhyle substitué sur un composé comportant au moins une fonction électrophile, caractérisé par le fait qu'il comprend :
a) un acide fluorocarboxylique de formule Ea - CF₂ - COOH où Ea représente un atome ou un groupe électroat-tracteur, au moins partiellement salifié par un cation organique ou minéral, le cation minéral étant choisi parmi le sodium, le potassium, le rubidium, le césium et le francium, et
b) un solvant aprotique polaire ;
par le fait que sa teneur en protons libérables portés par ses divers composants, y compris leurs impuretés, est au plus égale à la moitié de la concentration molaire initiale en ledit acide fluorocarboxylique,
et en ce que la teneur en cuivre est inférieure à 1 000 ppm par rapport à la teneur initiale en ledit acide fluorocarboxylique.

Les fonctions électrophiles susceptibles de réagir avec le réactif de la présente invention sont les fonctions qui réagissent habituellement avec les organométalliques et seront détaillées par la suite.

Plus la teneur en protons libérables dans le réactif sera faible, moins il y aura de risque de réaction parasite et meilleur sera le rendement.

Ainsi, il est préférable que, dans le réactif, la teneur en atomes d'hydrogène labiles soit au plus égale à 10%, de préférence à 1% (en moles), par rapport à la teneur initiale en ledit acide fluorocarboxylique.

La principale impureté, porteuse d'atomes d'hydrogène labiles, est en général l'eau qui est susceptible de libérer jusqu'à deux atomes d'hydrogène par molécule.

D'une manière générale il est préférable d'utiliser des réactifs et des solvants soigneusement déshydratés, de manière que la teneur pondérale en eau du réactif soit au plus égale à 1 pour 1000, par rapport à la masse totale du réactif.

En fonction de l'ensemble des conditions réactionnelles, de telles teneurs en eau peuvent être satisfaisantes, mais dans certains cas, il peut être intéressant d'opérer à des niveaux plus bas, par exemple de l'ordre de 1 pour 10 000.

Toutefois, il n'est pas nécessairement indispensable d'éliminer la totalité de l'eau et un rapport molaire eau/acide fluorocarboxylique inférieur à 10 % peut être toléré.

Par ailleurs, il a pu être montré que d'autres éléments, à savoir les éléments de transition ayant deux états de valence stables, tels le cuivre, pouvaient n'être pas fastes, voire pouvaient être néfastes.

Bien que ce réactif selon l'invention ne nécessite pas de catalyseur, de tels éléments métalliques peuvent être présents en tant qu'impuretés apportées notamment par le solvant.

Ainsi, il est préférable que la teneur molaire en ces éléments soit inférieure à 1000, avantageusement à 100, de préférence à 10 ppm par rapport à la teneur initiale en ledit acide fluorocarboxylique.

Egalement, bien qu'il ait été de nombreuses fois préconisé d'utiliser avec l'acide perfluoroacétique des éléments de la colonne VIII de la classification périodique des éléments, pour favoriser certains substrats et favoriser certains types de réaction, cela s'est révélé particulièrement néfaste pour la réaction visée ci-dessus. C'est pourquoi il est préférable d'utiliser des réactifs ne contenant pas de métaux de la colonne VIII, notamment des métaux de la mine du platine qui est le groupe constitué du platine, de l'osmium, de l'iridium, du palladium, du rhodium et du ruthénium.

Dans la présente description, il est fait référence au supplément au bulletin de la Société Chimique de France numéro 1, janvier 1966, où une classification périodique des éléments a été publiée.

Ainsi il est préférable que la teneur en métaux de la mine du platine, voire en métaux de la colonne VIII, soit inférieure à 100 ppm, avantageusement à 10 ppm, de préférence à 1 ppm. Ces valeurs s'entendent par rapport à l'acide fluorocarboxylique de départ et sont exprimées en moles.

D'une manière plus générale et plus empirique, on peut indiquer que ces deux catégories de métaux, à savoir les éléments de transition à deux états de valence et les éléments de la colonne VIII, doivent être présents dans le réactif à un niveau de concentration globale au plus égal à 1000 ppm molaires, de préférence à 10 ppm molaires.

On notera que les différents métaux présents à un tel niveau de concentration globale sont en quantité extrêmement faible et, à cet égard, ils ne jouent aucune rôle catalytique. Leur présence n'améliore pas la cinétique de la réaction, voire lui est néfaste lorsqu'ils sont présents en trop grande quantité.

L'utilisation, en plus des composants de réactifs précités, de fluorure de métal alcalin ou de fluorure d'ammonium quaternaire, habituellement présents dans les systèmes réactifs utilisant des carboxylates fluorés, ne s'est pas révélée néfaste, mais elle s'est révélée de peu d'intérêt, en raison du fait qu'elle produit des effluents salins difficiles à traiter.

On note cependant que la présence de fluorures dans le milieu a tendance à limiter la transformation de l'acide fluorocarboxylique mais à réduire les réactions parasites.

Cet effet a tendance à être d'autant plus important que le contre-cation du fluorure est volumineux. Des cations qui peuvent être envisagés sont les cations de métaux alcalin de rang supérieur à celui du sodium, en particulier potassium ou césium, ou bien les ions de type "onium", à savoir des cations formés par les éléments des colonnes V B et VI B (tels que définis dans le tableau de classification périodique des éléments publiés au supplément au Bulletin de la Société Chimique de France en janvier 1966), avec 4 ou 3 chaînes hydrocarbonées.

Parmi les oniums dérivant d'éléments de la colonne V B, les réactifs préférés sont les tétraalcoyl ou tétraaryl ammonium ou phosphonium. Le groupe hydrocarboné comporte avantageusement de 4 à 12 atomes de carbone, de préférence de 4 à 8 atomes de carbone. Les oniums dérivant de la colonne VI B sont de préférence dérivés d'éléments de numéro atomique supérieur à celui de l'oxygène.

Malgré les inconvénients qui ont été cités plus haut, la teneur en ions fluorure est un paramètre que l'on pourra considérer. Il peut toutefois être préférable de limiter cette teneur, en particulier la teneur initiale, afin de faciliter le traitement final du milieu réactionnel.

Ainsi il est avantageux que la teneur en fluorure, que l'on qualifie de ionique, c'est-à-dire susceptible d'être ionisé dans le milieu polarisant du réactif, soit au plus égale à la concentration molaire initiale en ledit sel d'acide fluorocarboxylique, avantageusement à la moitié, de préférence au quart.

Ainsi qu'on l'a mentionné ci-dessus, le solvant joue un rôle important dans la présente invention et doit être aprotique polaire et comporter très peu d'impuretés porteuses d'hydrogène acide.

Il est ainsi préférable que le solvant aprotique polaire utilisable ait un moment dipolaire significatif. Ainsi, sa constante diélectrique relative e est avantageusement au moins égale à environ 5 (les zéros de position ne sont pas considérés comme des chiffres significatifs dans la présente description à moins qu'il en soit précisé autrement). De préférence l'∈ est inférieur ou égal à 50 et supérieur ou égal à 5, et est notamment compris entre 30 et 40.

On préfère en outre que les solvants de l'invention soient susceptibles de bien solvater les cations, ce qui peut être codifié par l'indice donneur D de ces solvants. Il est ainsi préférable que l'indice donneur D de ces solvants soit compris entre 10 et 30. Ledit indice donneur correspond au ΔH (variation d'enthalpie), exprimé en kilo calorie par mole, de l'association dudit solvant aprotique polaire avec le pentachlorure d'antimoine.

Selon la présente invention, il est préférable que le réactif ne présente pas d'hydrogène acide sur le ou les solvants polaires qu'il utilise. En particulier lorsque le caractère polaire du ou des solvants est obtenu par la présence de groupes électroattracteurs, il est souhaitable qu'il n'y ait pas d'hydrogène en alpha de la fonction électroattractrice.

De manière plus générale, comme tous les composants du réactifs, il est préférable que le pKa correspondant à la première acidité du solvant soit au moins égal à environ 20 ("environ" soulignant que seul le premier chiffre est significatif), avantageusement au moins égal à environ 25, de préférence compris entre 25 et 35.

Le caractère acide peut également être exprimé par l'indice accepteur A du solvant, tel qu'il est défini par Reichardt, "Solvents and solvent effects in Organic Chemistry", 2ème édition, VCH (RFA), 1990, pages 23-24. Avantageusement, cet indice accepteur A est inférieur à 20, en particulier inférieur à 18.

Il est préférable que ledit acide ou sel d'acide fluorocarboxylique soit au moins partiellement (au moins 10 % en mol), de préférence complètement, soluble dans le milieu constituant le réactif.

Les solvants donnant de bons résultats peuvent être notamment des solvants de type amide. Parmi les amides, on comprend aussi les amides à caractère particulier comme les urées tétrasubstituées y compris les urées tétrasubstituées cycliques, notamment à 5 ou 6 chaînons, par excemple la DMPU (diméthylpropylenylurée ou 1,3-diméthyl-3,4,5,6-tétrahydro-2(1H)pyrimidinone) et la DMEU (diméhyléthylenylurée), ou 1,3-diméthyl-2-imidazolidinone et les lactames monosubstitués. Les amides sont de préférence substitués (disubstitués pour les amides ordinaires). On peut citer par exemple les dérivés de pyrrolidone, tels que la N-méthylpyrrolidone, ou encore le N,N-diméthylformamide, ou le N,N-diméthylacétamide.

Sont également avantageux des solvants tels que la 1,3-diméthyl-3,4,5,6-tétrahydro-2(1H)pyrimidinone (DMPU) ou le benzonitrile.

Une autre catégorie particulièrement intéressante de solvants est constituée par les éthers, qu'ils soient symétriques ou non symétriques, qu'ils soient ouverts ou non. Dans la catégorie des éthers doivent être incorporés les différents dérivés des éthers de glycol tels que les différents glymes, le diglyme par exemple.

Dans l'acide fluorocarboxylique du constituant a) du réactif de l'invention, l'entité Ea qui exerce un effet électroattracteur sur l'atome de carbone difluoré est de préférence choisie parmi les groupes fonctionnels dont la constante de Hammett σₚ est au moins égale à 0,1. Il est en outre préférable que la composante inductive de σₚ, σᵢ, soit au moins égale à 0,2, avantageusement à 0,3. A cet égard, on se référera à l'ouvrage de March, "Advanced Organic Chemistry", troisième édition, John Wiley and Son, pages 242 à 250, et notamment au tableau 4 de cette section.

Plus particulièrement, l'entité électroattractrice peut être choisie parmi les atomes d'halogène, de préférence légers, notamment de chlore et de fluor. L'acide fluorocarboxylique correspondant est un acide halogénofluoroacétique de formule (1)
X - CF₂ - COOH où X est un atome d'halogène, avantageusement léger (chlore ou fluor).

Ea peut également être avantageusement choisie parmi les groupements nitriles (avec le risque, comme réaction parasite, d'une α-élimination), carbonylés, sulfonés et perfluoroalcoylés. Des acides fluorocarboxyliques de ce type qui peuvent être utilisés répondent à la formule (2) R - G - CF₂ - COOH où R - G représente un groupe nitrile ou bien G représente 〉C = O, 〉S = 0, ou -(CF₂)ₙ- où n est supérieur ou égal à 1, et R représente un résidu organique, voire minéral, indifférent, de préférence un radical organique tel que aryle, alcoyle, ou aralcoyle, éventuellement substitué. R peut également représenter un support solide organique, tel qu'une résine, ou minéral.

Dans le cas où G représente un groupe perfluoroalkylène - (CF₂)ₙ -, n est avantageusement compris entre 1 et 10, de préférence entre 1 et 5. Toujours dans ce cas, R peut également représenter un atome d'halogène, notamment le fluor.

De manière générale, sauf dans le cas où l'acide fluorocarboxylique est un polymère, le nombre total d'atomes de carbone de l'acide fluorocarboxylique, n'excède avantageusement pas 50.

Les contre cations susceptibles de former un sel avec ledit acide fluorocarboxylique sont avantageusement volumineux. Ainsi, on préfère des sels alcalins où le métal alcalin est choisi parmi le sodium, le potassium, le rubidium, le césium et le francium. De préférence, ledit métal est d'une période dont le rang est au moins égal à celle du sodium, avantageusement à celle du potassium. On préfère également des sels d'ammonium quaternaire.

Il est également possible d'améliorer la réaction en utilisant des cations qui sont, soit naturellement volumineux comme les cations ammonium quaternaire ou phosphonium quaternaire, ou rendus volumineux par l'addition d'agents chélatants ou de préférence cryptands, tels que par exemple les éthers couronne ou les dérivés qui sont à la fois aminés et oxygénés.

Les agents chélatant ou séquestrants alors utilisables sont avantageusement choisis d'une part parmi les amines et d'autre part parmi les éthers dont les molécules comportent au moins une autre fonction éther.

Ainsi, les agents séquestrants utilisables sont avantageusement choisis de manière qu'ils comportent, ou bien au moins une fonction amine ; ou bien une fonction éther et au moins une fonction amine et/ou éther pour former un complexant avantageusement au moins bidenté, de préférence tridenté, les fonctions éther et/ou amine étant séparées par au moins 1, avantageusement 2 atomes et par au plus 4, avantageusement au plus 3 atomes, en général de carbone.

Lorsque les atomes réputés assurer la coordination sont reliés entre eux par 2 branches formant ainsi un cycle, il est préférable qu'une branche au moins comporte au moins 3 chaînons avantageusement 4 chaînons et l'autre au moins 2, avantageusement 3 chaînons.

L'encombrement et la mobilité doivent être tels que les bi, tri ou polydentés soient complexants. Tel n'est pas le cas du 1,4 diaza (2,2,2) bicyclo octane.

D'une manière générale, on peut quantifier cette contrainte en indiquant que les systèmes bicycliques obtenus par pontage d'un cycle (qui sont en fait tricycliques), et présentant un nombre de chaînons au plus égal à 8, surtout lorsque les têtes de pont sont les atomes assurant la coordination, du type diaza bicyclo-octane, heptane et inférieur et dans une moindre mesure nonane sont à éviter.

D'une manière plus générale, il est avantageux d'éviter tout système bicyclique :
- dont les têtes de pont sont des atomes prévus pour assurer la coordination
   et
- dont 2 des branches ont, compte non tenu des têtes de pont, une longueur de chaînons au plus égal à 2, de préférence au plus égal à 3 lorsque la troisième branche présente une longueur, exprimée en chaînon, inférieur à 7.

Le caractère bidenté au moins avec de préférence au moins une fonction amine est nécessaire pour le phosgène et dérivés, mais non pour l'halogénure d'oxalyle et équivalents.

On peut citer comme particulièrement intéressantes au moins 3 classes d'agents complexants comprenant les amines tertiaires oxygénées ; les polyéthers oxygénés ou soufrés, cycliques ou macrocycliques ; les cryptands.

La première classe est constituées des agents séquestrants de formule générale :

N- [-CHR₁-CHR₂-O-(CHR₃-CHR₄-O)ₙ-R₅]₃ (I)

dans laquelle n est un nombre entier supérieure ou égal à 0 et inférieur ou égal à 10 environ (0 < n < 10), R₁, R₂, R₃ R₄ identiques ou différents représentent un atome d'hydrogène ou un radical alcoyle ayant de 1 à 4 atomes de carbone et R₅ représente un radical alcoyle ou cycloalcoyle ayant de 1 à 12 atomes de carbone, un radical phényle ou un radical de formule -CₘH₂ₘC₆H₅, ou CₘH₂ₘ₊₁-C₆H₅, m étant compris entre 1 et environ 12.

La deuxième classe d'agents complexants est constituée des polyéthers cycliques, de préférence macrocycliques, ayant de 6 à 30 atomes dans le cycle et de préférence de 15 à 30 atomes dans le cycle et constitués de 2 à 10, de préférence de 4 à 10 unités -O-X dans lesquelles X est soit -CHR₆-CHR₇- soit -CHR₆-CHR₈-CR₉R₇, R₆, R₇, R₈ et R₉ identiques ou différents étant un atome d'hydrogène ou un radical alcoyle ayant de 1 à 4 atomes de carbone, un des X pouvant être -CHR₆-CHR₈-CR₉R₇- quand les unités -O-X- comprennent le groupement -O-CHR₆-CHR₇.

La troisième classe d'agents complexants est constituée des composés décrits dans la demande de brevet EP 0423008 page 3 ligne 29 à page 6 ligne 45.

Des sels d'acides perfluorocarboxyliques peuvent être avantageusement utilisés, tels que les trifluoroacétate, perfluoropropionate et perfluorobutyrate de métal alcalin, notamment de potassium.

On note que l'utilisation de séquestrants du type éthers couronnes, dans des solvants qui sont relativement peu polaires (moins polaires que le DMF), accélère de façon marquée la transformation de l'acide fluorocarboxylique de départ.

De tels séquestrants pourront être utilisés avantageusement à raison de 5 à 100 % en mole, notamment de 5 à 25 % en mole par rapport à la teneur initiale en acide fluorocarboxylique.

Toutefois, certaines associations avec les autres partenaires du milieu réactionnel, notamment certains solvants, pourront avoir un effet moins favorable en ce qui concerne la stabilité du produit d'arrivée, et ne seront donc pas considérées comme avantageuses.

Un autre but de la présente invention est de fournir un procédé de synthèse d'un dérivé organique comportant un groupement difluorométhylène, qui utilise le réactif selon la présente invention.

Ce but est atteint :
a) par la mise en présence dudit réactif avec un composé comportant au moins une fonction électrophile, et
b) par le chauffage du mélange résultant à une température comprise entre 100°C et 200°C, de préférence entre 110 et 150°C, et ce, pendant une durée d'au moins une demi-heure, avantageusement d'au moins une heure, et d'au plus une journée, avantageusement de moins de 20 heures.

La mise en présence, ou en contact du réactif avec le substrat peut être progressive ou non. En particulier on peut attendre que l'un des deux soit à la bonne température pour introduire l'autre. Cette introduction peut être progressive ou non. On peut couler le réactif dans le substrat ou réciproquement. On peut introduire le fluorocarboxylate et le substrat à la fois simultanément et progressivement dans le solvant.

Le réactif de l'invention réagit selon l'invention avec un composé électrophile, comportant un atome électrophile, cet atome pouvant être un atome de carbone ou un hétéroatome, par exemple le soufre, le sélénium ou le tellure. Il réagit avantageusement avec des composés hydrocarbonés sur un atome de carbone électrophile n'appartenant pas à un système aromatique.

Selon un premier aspect de l'invention, le réactif réagit de préférence avec des composés comportant un atome électrophile, avantageusement un hétéroatome électrophile, lié à un atome d'halogène ou à un groupement pseudohalogène pour réaliser la substitution dudit halogène ou pseudohalogène en une étape.

La réaction marche d'autant mieux que, par opposition avec une SN2, l'on passe par un intermédiaire réactionnel provenant d'une addition sur une liaison multiple ou sur un doublet.

Lorsque l'atome électrophile est un atome de soufre, on peut citer la réaction avec :
- les dérivés halogénés ou pseudohalogénés de composés organiques du soufre, notamment les halogénures de sulfényle, de sulfinyle ou de sulfonyle, où l'atome d'halogène ou le groupement pseudohalogène est substitué au cours de la réaction par un groupement difluorométhyle substitué ;
- des disulfures,par exemple des aryldisulfures éventuellement substitués, où la liaison S-S est rompue et remplacée par un groupement difluorométhyle substitué ; des disulfures appropriés peuvent être notamment des aryldisulfures en C₅-C₁₀, éventuellement substitués par un groupe alkyle en C₁-C₁₀, alcoxy en C₁-C₁₀, nitro ou par un ou plusieurs (≤ 3) atome(s) d'halogène ;
- les composés de type thiocyanate où le groupe cyano est substitué au cours de la réaction par un groupement difluorométhyle substitué ; des thiocyanates préférés sont les thiocyanates d'aryle en C₅-C₁₀, y compris d'alcoylaryle, et thiocyanates d'alcoyle en C₁-C₁₀, y compris d'aralcoyle.

Dans les composés ci-dessus, l'atome d'halogène peut être choisi parmi les atomes d'iode, de brome, de chlore et de fluor. Un groupement "pseudohalogène" est un groupe qui, partant, sous forme anionique, présente un acide associé dont le pKa est inférieur à 4, de préférence à 3, en particulier à 0.

On préfère les groupes dont l'acide associé présente une acidité (mesurée par la constante de Hammett) au moins égale à celle de l'acide acétique, avantageusement à celle des acides sulfoniques, ou des acides trihalogénés. Un des pseudohalogènes typiques est un groupe perfluoroalcanesulfonyloxyle qui libère un perfluoroalcanesulfonate. Des groupes pseudohalogènes préférés peuvent être choisis parmi les groupe tosylate (p-toluènesulfonyloxyle), mésylate (méthylsulfonyloxyle), trifluorométhylsulfonyloxyle ou trifluoroacétoxyle. On peut également considérer le groupe acétate comme un tel groupe partant.

Selon un second aspect, le réactif réagit également avantageusement sur un composé choisi parmi les composés carbonylés de type cétone, aldéhyde, halogénure d'acide ou ester activé, anhydride, en réalisant une addition sur la fonction carbonyle. On pourra citer à titre d'exemples préférentiels et non limitatifs des aldéhydes aromatiques, de préférence en C₅-C₁₀, où le noyau aromatique peut éventuellement être substitué par un groupe alkyle en C₁-C₁₀, alcoxy en C₁-C₁₀, nitro ou par un atome d'halogène ; des cétones cycliques comme la cyclohexanone ; des cétones non énolisables activées par un groupe donneur, comme la trifluorométhylacétophénone; des anhydrides aromatiques, comme l'anhydride benzoïque.

Lorsqu'il y a un risque de réaction entre le substrat et le fluorocarboxylate, alors il peut être préférable de n'introduire le substrat ou le fluorocarboxylate que dans les conditions de décarboxylation dudit carboxylate (voir plus haut les conditions de mise en oeuvre).

Le produit de la réaction est en général dans ce cas un alcool (par exemple sous forme d'alcoolate) dont l'atome de carbone porteur de la fonction hydroxyle est substitué par un groupement difluorométhyle substitué. Ce produit peut éventuellement réagir ensuite avec le réactif ou avec le produit de départ suivant les conditions réactionnelles.

De manière générale, la quantité de réactif engagé dans le procédé de l'invention sera fixée de manière connue en soi selon la fonctionnalité du composé électrophile.

Il convient de signaler que le produit issu de la décomposition de l'acide fluorocarboxylique peut réagir sur lui-même s'il comporte une des fonctions susceptibles de réagir telles que celles mentionnées ci-dessus.

On pourra noter que des composés à fonction électrophile se présentant sous forme liquide sont susceptibles d'être utilisés en tant que solvant selon la présente invention dans la mesure où ils sont aprotiques. On peut ainsi réaliser avantageusement la réaction de la présente invention en mettant en présence
a) un sel d'acide fluorocarboxylique tel que défini précédemment et
b) un composé comportant au moins une fonction électrophile agissant à la fois comme solvant et comme substrat de la réaction.

Lors de la mise en oeuvre du réactif selon l'invention avec un substrat comportant au moins une fonction électrophile,il est important que ce dernier perturbe le moins possible les conditions décrites précédemment.

Ainsi, il est préférable d'utiliser un substrat suffisamment déshydraté, ou qui ne comporte pas d'hydrogène acide arrachable par des bases fortes ni d'impuretés néfastes c'est-à-dire, d'une manière générale, qui satisfasse les mêmes contraintes que celles exposées pour le réactif.

On a pu constater que, toutes choses étant égales par ailleurs, le rendement en le dérivé organique visé dépend du degré d'avancement de la réaction et que l'on peut obtenir un rendement final très faible malgré une conversion importante des réactifs. Sans vouloir être lié à une quelconque théorie scientifique, il semble que tout se passe comme s'il y avait une cinétique de formation et une cinétique de dégradation des produits obtenus.

Pour éviter une dégradation trop importante du produit final, et donc assurer une bonne sélectivité de la réaction, il est préférable de ne pas chercher à convertir complètement l'acide fluorocarboxylique de départ. L'avancement de la réaction peut être contrôlé par le taux de transformation (TT) de l'acide qui est le rapport molaire de la quantité d'acide disparue sur la quantité d'acide initiale dans le milieu réactionnel, ce taux étant calculé aisément après dosage de l'acide restant dans le milieu.

De manière avantageuse, on ne conduira la réaction que jusqu'à l'obtention d'un taux de transformation de 40 à 80 %, de préférence de 50 à 70 %, puis on séparera les produits réactionnels. Il est possible d'atteindre ainsi une sélectivité de l'ordre de 80 % exprimée par le rapport molaire produit recherché/acide fluorocarboxylique transformé.

Pour se placer dans des conditions réactionnelles optimales, il est possible de limiter le taux de transformation en agissant à la fois sur la durée de la réaction, la nature du solvant et la présence d'additifs qui ont tendance à limiter cette transformation, tels que des ions fluorures par exemple. La cinétique de la réaction dépend, en outre, des partenaires réactionnels (acide fluorocarboxylique et réactif électrophile) et le temps de réaction approprié pourra aisément être adapté au cas par cas en fonction de cette cinétique.

Une fois atteint le taux de transformation désiré, le mélange réactionnel peut être traité de façon connue en soi pour séparer le produit obtenu, les produits de départ pouvant être recyclés pour produire une quantité supplémentaire du dérivé organique visé.

Le cas échéant, on pourra mettre en oeuvre pour la séparation une réaction chimique supplémentaire permettant de convertir le produit désiré en un dérivé plus volatil et facilement distillable.

Les exemples suivants illustrent l'invention.

### Exemple 1 : Synthèse de l'alcool 1-trifluorométhylbenzylique.

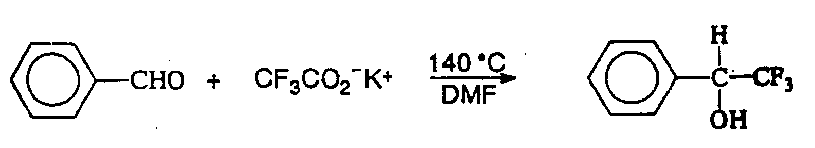

Sous atmosphère d'azote, on mélange dans 26 g de DMF anhydre 4,98 g (32,7 mmol) de trifluoroacétate de potassium et 2 g (18,8 mmol) de benzaldéhyde.

Le rapport molaire du trifluoroacétate au benzaldéhyde est de 1,7.

Le mélange obtenu est transféré dans un réacteur en Hastelloy de 50 ml. Le réacteur étant fermé, on chauffe le mélange à 104°C pendant 3h30.

Après refroidissement à 5°C le brut réactif est soutiré, dilué dans CH₂Cl₂ et lavé à l'eau.

La phase organique est séchée puis dosée par chromatographie en phase gazeuse.

Le taux de transformation (TT) du benzaldéhyde est de 50 % (en nombre de moles de benzaldéhyde transformé par rapport au nombre de moles de benzaldéhyde initial) et le rendement réel (RR) en alcool 1-trifluorométhyl benzylique est de 20 %.

### Exemple 2.

La réaction entre le trifluoroacétate de potassium et le benzaldéhyde est conduite comme dans l'exemple 1 en remplaçant le DMF par de la NMP (N-méthyl pyrrolidone).

7,6 g de CF₃CO₂⁻K⁺ (50 mmol) et 3,2 g de benzaldéhyde (30 mmol) sont dissous dans 40 g de NMP.

La teneur en eau du milieu et inférieur à 4 % en moles par rapport au trifluoroacétate.

Le mélange est chauffé à 140°C pendant 3h30.

Le traitement et le dosage du brut réactionnel conduit comme dans l'exemple 1 donne
Taux de transformation du benzaldéhyde = 55 %
Rendement en alcool 1-trifluoro methyl benzylique = 15 %

### Exemple 3.

La réaction entre le trifluoroacétate de potassium et le benzaldéhyde est conduite comme dans l'exemple 1, le DMF étant remplacé par de l'acétonitrile. 2 g de benzaldéhyde et 4,75 g de trifluoroacétate de potassium sont dissous, dans 25 ml de CH₃CN.

Le mélange est chauffé à 140°C pendant 3h30.

Après traitement et dosage du brut réactionnel on obtient
Taux de transformation du benzaldéhyde = 53 %
Rendement en alcool 1-trifluoro methyl benzylique = 2,5 %

Le produit principal formé dans cette réaction est le cinnamonitrile (isomères Z et E).

Le cinnamonitrile est formé par condensation de l'anion de l'acétonitrile sur le benzaldéhyde, suivie d'une déshydratation.

Cet exemple montre que le solvant à utiliser ne doit pas avoir de protons trop acides.

### Exemple 4.

Dans les conditions de l'example 1 on réalise la réaction entre le trifluoroacétate de potassium (5,05 g ; 32,7 mmol) et du parafluorobenzaldéhyde (2,5 g ; 20,2 mmol) dans 25 ml de DMF.

Le mélange est chauffé à 140°C pendant 4h00.

Après traitement le dosage par chromatographie en phase gazeuse (CPV) donne
TT p-fluorobenzaldehyde = 75 %
RR alcool 1-trifluorométhyl (p-fluorobenzylique) ≤ 2 %

Le produit majoritaire correspond à l'addition du trifluorométhyle carbinolate intermédiairement formé sur le parafluorobenzaldéhyde :

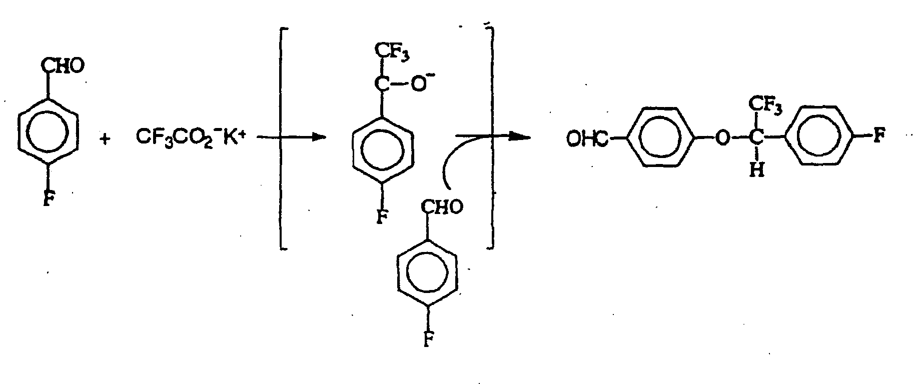

Cette réaction montre que lorsque l'électrophile utilisé possède plusieurs fonctions réactives des réactions secondaires peuvent se produire.

### Exemple 5.

Un mélange constitué de 1,43 g de CF₃CO₂⁻K⁺ (9,44 mmol) et 0,55 g de cyclohexanone (5,6 mmol) dilué dans 6,4 g de DMF est chauffé à 140°C pendant 5h30. L'analyse par CPV du brut réactionnel après hydrolyse donne

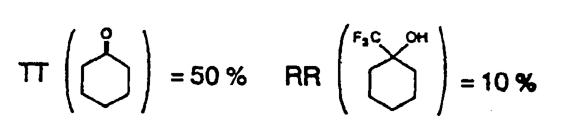

Les produits majoriatires formés correspondent aux produits de condensation de la cyclohexanone sur elle même suivie d'une deshydratation :

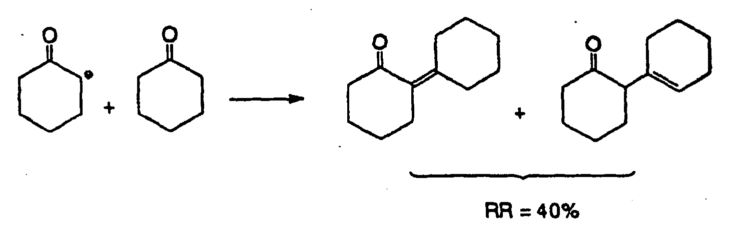

Cette réaction montre que lorsque l'élecrophile possède une fonction enolisable, des réactions secondaires peuvent avoir lieu.

### Exemple 6 : Réaction de la trifluorométhylacétophénone avec CF3CO2K.

Un mélange de CF₃CO₂⁻K⁺ (0,87 g ; 5,7 mmol) et 0,62 g (3,56 mmol) de trifluorométhylacétophénone dissous dans 6,5 g de DMF est chauffé à 140°C pendant 5h30.

Après refroidissement et hydrolyse l'analyse par CPV du milieu réactionnel donne

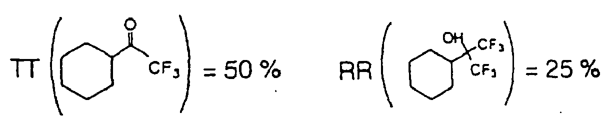

La même réaction peut être faite dans la NMP au lieu du DMF.

### Exemple 7 : Réaction entre l'anhydride benzoique et le trifluoroacétate de potassium.

Un mélange de 0,81 g (5,32 mmol) de CF₃CO₂⁻K⁺ et 0,7 g (3,1 mmol) d'anhydride benzoique dans 6,15 g de DMF est chauffé à 140°C pendant 5h30. Après hydrolyse l'analyse par CPV du milieu réactionnel donne

TT ((ΦCO)₂O) = 100 %

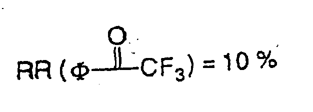

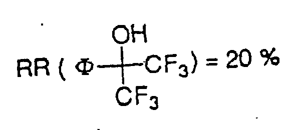

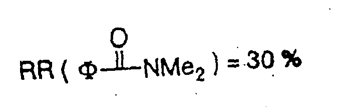

Le bis trifluorométhylcarbinol 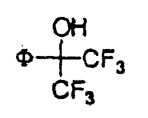 vient de la trifluorométhylation de la trifluorométhylacétophénone formée intermédiairement :

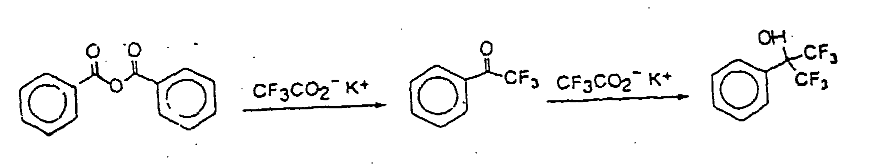

Le N-N-diméthyl benzamide provient d'une réaction de dégradation du DMF qui produit de la N-N-diméthylamine, celle-ci réagissant avec l'anhydride benzoïque :

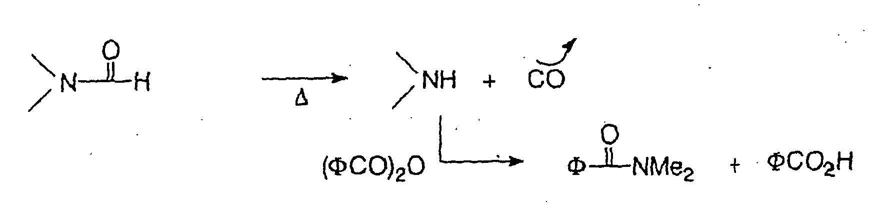

La réaction entre l'anhydride benzoïque et CF₃CO₂⁻K⁺ peut également être conduite dans le NMP.

### Exemple 8 : Réaction entre le diphényldisulfure C₆H₅SSC₆H₅ et CF₃Co₂⁻K⁺.

Un mélange de 0,83 g (5,46 mmol) de CF₃CO₂⁻K⁺ 0,6 g (2,75 mmol) de diphényldisulfure dans 6,2 g de DMF est chauffé à 140°C pendant 6h00.

L'analyse du milieu réactionnel (après hydrolyse) par CPV et RMN ¹⁹F donne
TT (C₆H₅SSC₆H₅) = 67 %
RR (C₆H₅SCF₃) = 84 %.

La réaction peut être conduite de la même manière dans la NMP.

### Contre-exemple.

En procédant comme à l'exemple 8 mais avec une addition supplémentaire de 20 % molaire de CuI par rapport au CF₃CO₂⁻K⁺ initial (5,46 mmol), on obtient une inhibition totale de la réaction de trifluorométhylation du diphényldisulfure.

### Exemple 9 : Réaction entre le di(4-nitrophényl)disulfure et CF₃CO₂⁻K⁺.

En reproduisant le protocole de l'exemple 8 avec un mélange de 0,82 g (5,39 mmol) de CF₃CO₂⁻K⁺, 0,83 g (2,7 mmol) de di(4-nitrophényl)disulfure dans 7 g de DMF on obtient un brut réactionnel contenant, outre le di(4-nitrophényl)disulfure de départ, également du 4-nitro thiofluorométhylbenzène

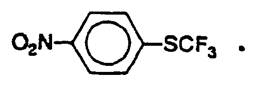

### Exemple 10 : Réaction du thiocyanate de benzyle C₆H₅CH₂SCN avec CF₃CO₂⁻K⁺.

Le mélange de 0,67 g (4,45 mmol de CF₃CO₂⁻K⁺ 0,42 g (2,8 mmol) de thiocyanate de benzyle dans 5 g de DMF est chauffé à 140°C pendant 3h00.

Après hydrolyse l'analyse CPV du brut réactionnel donne :
TT (C₆H₅-CH₂SCN) = 100 %
RR (C₆H₅-CH₂SCF₃) = 36 %

La réaction peut être conduite dans la NMP de façon similaire.

## Revendications

1. Réactif nucléophile **caractérisé en ce qu'**il comprend :
a) un acide fluorocarboxylique de formule Ea - CF₂ - ÇOOH où Ea représente un atome ou un groupe électroattracteur, au moins partiellement salifié par un cation organique ou minéral, le cation minéral étant choisi parmi le sodium, le potassium, le rubidium, le césium et le francium, et
b) un solvant aprotique polaire ;
**en ce que** la teneur en protons libérables portés par ses divers composants, y compris leurs impuretés, est au plus égale à la moitié de la concentration molaire initiale dudit acide fluorocarboxylique ; et
**en ce que** la teneur en cuivre est inférieure à 1 000 ppm par rapport à la teneur initiale en ledit acide fluorocarboxylique.

2. Réactif selon la revendication 1 ou 2, **caractérisé par le fait que** ladite teneur en protons est au plus égale à 10 % de la concentration molaire initiale en ledit sel d'acide fluorocarboxylique.

3. Réactif selon l'une quelconque des revendications précédentes, **caractérisé en ce que** sa teneur en eau est inférieure à 10 % de la concentration molaire en ledit acide fluorocarboxylique.

4. Réactif selon l'une quelconque des revendications précédentes, **caractérisé en ce que** sa teneur en éléments de transition présentant au moins deux états de valence stables est inférieure à 1000 ppm molaires, par rapport audit sel d'acide fluorocarboxylique.

5. Réactif selon l'une quelconque des revendications précédentes, **caractérisé par le fait que** sa teneur en éléments de la colonne VIII de la classification périodique des éléments est inférieure à 100 ppm molaires, par rapport audit sel d'acide fluorocarboxylique.

6. Réactif selon l'une quelconque des revendications précédentes, **caractérisé par le fait que** sa teneur, exprimée en équivalent, en fluorure ionique est au plus égale à la concentration molaire initiale dudit sel d'acide fluorocarboxylique.

7. Réactif selon l'une quelconque des revendications précédentes, **caractérisé par le fait que** l'indice donneur dudit solvant aprotique polaire est compris entre 10 et 30.

8. Réactif selon l'une quelconque des revendications précédentes, **caractérisé par le fait que** l'indice accepteur dudit solvant est inférieur à 20.

9. Réactif selon l'une quelconque des revendications précédentes, **caractérisé par le fait que** le pKa correspondant à la première acidité dudit solvant est au moins égale à 20.

10. Réactif selon l'une quelconque des revendications précédentes, **caractérisé en ce qu'**il comprend un éther couronne séquestrant.

11. Réactif selon l'une quelconque des revendications précédentes, **caractérisé en ce que** ledit atome ou groupement électroattracteur est choisi parmi les groupes électroattracteurs dont la constante de Hammett σₚ est au moins égale à 0,1.

12. Réactif selon l'une quelconque des revendications précédentes, **caractérisé par le fait que** ledit acide est choisi parmi les composés de formule (1) X - CF₂ - COOH, où X représente un atome d'halogène, et les composés de formule (2) R - G - CF₂ - COOH, où R - G représente un groupe nitrile ou G représente C=O, S=O ou -(CF₂)ₙ- avec n supérieur ou égal à 1 et R représente un résidu organique ou minéral indifférent.

13. Réactif selon l'une quelconque des revendications précédentes, **caractérisé par le fait que** ledit acide ou sel d'acide fluorocarboxylique est complètement soluble dans le milieu réactif.

14. Réactif selon l'une quelconque des revendications précédentes, **caractérisé par le fait que** ledit sel d'acide est un sel d'ammonium quaternaire.

15. Réactif selon l'une quelconque des revendications précédentes, **caractérisé par le fait que** les solvants sont choisis parmi les amides N-disubstitués, y compris les urées tétrasubstituées et les lactames monosubstitués, et les éthers cycliques ou non, et le benzonitrile.

16. Réactif nucléophile selon la revendication 1, **caractérisé en ce que** ledit solvant comporte au moins une fonction électrophile.

17. Procédé de synthèse d'un dérivé comportant un groupement difluorométhylène, **caractérisé par le fait qu'**il comporte l'étape :
a) de mise en présence d'un réactif selon les revendications 1 à 16, avec un composé comportant au moins une fonction électrophile à l'exception des atomes de carbone électrophiles appartenant à un noyau aromatique, et
b) de chauffage du mélange résultant à une température comprise entre 100 et 200°C, pendant une durée comprise entre 1/2 heure et un jour.

18. Procédé selon la revendication 17, **caractérisé en ce que** le composé comportant au moins une fonction électrophile est un aldéhyde aromatique.

19. Procédé de synthèse d'un dérivé comportant un groupement difluorométhylène, **caractérisé par le fait qu'**il comporte l'étape de chauffage d'un réactif selon la revendication 2 à une température comprise entre 100 et 200°C, pendant une durée comprise entre 1/2 heure et un jour.

20. Procédé selon l'une quelconque des revendications 17 à 19, **caractérisé par le fait que** ledit composé à fonction électrophile est choisi parmi les dérivés halogénés ou pseudohalogénés de composés organiques du soufre ; les disulfures ; les thiocyanates ; et les composés carbonylés.

21. Procédé selon la revendication 20, **caractérisé par le fait que** ledit composé à fonction électrophile est un disulfure, notamment un aryldisulfure.

22. Procédé selon la revendication 21, **caractérisé en ce que** ledit composé est un aryldisulfure en C₅-C₁₀ éventuellement substitué par un groupe choisi parmi les groupes alkyle en C₁-C₁₀, alkoxy en C₁-C₁₀, nitro et halogéno.

23. Procédé selon la revendication 19 **caractérisé en ce que** le composé carbonylé est choisi parmi les cétones, aldéhydes, halogénures d'acide, esters activés, et anhydrides.

24. Procédé selon la revendication 20, **caractérisé en ce que** le dérivé halogéné du soufre est choisi parmi les halogénures de sulfényle, de sulfinyle et de sulfonyle.

25. Procédé selon l'une quelconque des revendications 17 à 20, **caractérisé par le fait que** ledit composé comportant au moins une fonction électrophile ne comporte pas d'hydrogène arrachable par une base forte.

26. Utilisation d'un réactif nucléophile comprenant :
a) un acide fluorocarboxylique de formule Ea - CF₂ - COOH où Ea représente un atome ou un groupe électroattracteur, au moins partiellement salifié par un cation organique ou minéral, le cation minéral étant choisi parmi le sodium, le potassium, le rubidium, le césium et le francium, et
b) un solvant aprotique polaire ;
dans lequel la teneur en protons libérables portés par ses divers composants, y compris leurs impuretés, est au plus égaie à la moitié de la concentration molaire initiale dudit acide fluorocarboxylique, et dans lequel la teneur en cuivre est inférieure à 1 000 ppm par rapport à la teneur initiale en ledit acide fluorocarboxylique, pour greffer un groupement difluorométhyle substitué sur un composé comportant au moins une fonction électrophile, à l'exception des atomes de carbone appartenant à un noyau aromatique.

27. Utilisation selon la revendication 26, **caractérisée en ce que** ledit solvant aprotique polaire est le composé portant au moins une fonction électrophile.

28. Procédé selon l'une quelconque des revendications 17 à 25,
**caractérisé en ce que** ledit composé comportant au moins une fonction électrophile joue le rôle de réactif et de solvant aprotique polaire.

## Claims

1. Nucleophilic reagent, **characterised in that** it comprises:
a) a fluorocarboxylic acid of formula Ea-CF₂-COOH where Ea denotes an electron-attracting atom or group, at least partially salified by an organic or inorganic cation, the inorganic cation being selected from among sodium, potassium, rubidium, caesium and francium, and
b) a polar aprotic solvent;
**in that** the content of releasable protons carried by its various components, including their impurities, is equal to not more than half the initial molar concentration of said fluorocarboxylic acid; and
**in that** the copper content is less than 1,000 ppm based on the initial content of said fluorocarboxylic acid.

2. Reagent according to claim 1 or 2, **characterised in that** said proton content is at most equal to 10% of the initial molar concentration of said fluorocarboxylic acid salt.

3. Reagent according to any one of the preceding claims, **characterised in that** its water content is less than 10% of the molar concentration of said fluorocarboxylic acid.

4. Reagent according to any one of the preceding claims, **characterised in that** its content of transition elements having at least two stable valency states is less than 1000 molar ppm, in relation to said fluorocarboxylic acid salt.

5. Reagent according to any one of the preceding claims, **characterised in that** its content of elements from column VIII of the Periodic Table of Elements is less than 100 molar ppm, in relation to said fluorocarboxylic acid salt.

6. Reagent according to any one of the preceding claims, **characterised in that** its content, expressed as equivalents, of ionic fluoride is at most equal to the initial molar concentration of said fluorocarboxylic acid salt.

7. Reagent according to any one of the preceding claims, **characterised in that** the donor index of said polar aprotic solvent is between 10 and 30.

8. Reagent according to any one of the preceding claims, **characterised in that** the acceptor index of said solvent is less than 20.

9. Reagent according to any one of the preceding claims, **characterised in that** the pKa corresponding to the first acidity of said solvent is equal to at least 20.

10. Reagent according to any one of the preceding claims, **characterised in that** it comprises a sequestering crown ether.

11. Reagent according to any one of the preceding claims, **characterised in that** said electron-attracting atom or group is selected from among the electron-attracting groups having a Hammett constant σₚ of at least 0.1.

12. Reagent according to any one of the preceding claims, **characterised in that** said acid is selected from among the compounds of formula (1) X - CF₂ - COOH, where X denotes a halogen atom, and the compounds of formula (2) R - G - CF₂ - COOH, where R - G denotes a nitrile group or G denotes C=0, S=0 or -(CF₂)ₙ- where n is greater than or equal to 1 and R denotes an inert organic or inorganic residue.

13. Reagent according to any one of the preceding claims, **characterised in that** said fluorocarboxylic acid or fluorocarboxylic acid salt is completely soluble in the reactive medium.

14. Reagent according to any one of the preceding claims, **characterised in that** said acid salt is a quaternary ammonium salt.

15. Reagent according to any one of the preceding claims, **characterised in that** the solvents are selected from among the N-disubstituted amides, including the tetrasubstituted ureas and monosubstituted lactams, and cyclic or non-cyclic ethers, and benzonitrile.

16. Nucleophilic reagent according to claim 1, **characterised in that** the said solvent comprises at least one electrophilic function

17. Process for synthesising a derivative comprising a difluoromethylene group, **characterised in that** it comprises the step of:
a) bringing a reagent according to claims 1 to 16 into contact with a compound containing at least one electrophilic function, with the exception of the electrophilic carbon atoms belonging to an aromatic nucleus, and
b) heating the resulting mixture to a temperature of between 100 and 200°C for a period of between half an hour and one day.

18. Process according to claim 17, **characterised in that** the compound comprising at least one electrophilic function is an aromatic aldehyde.

19. Process for synthesising a derivative comprising a difluoromethylene group, **characterised in that** it comprises the step of heating a reagent according to claim 2 to a temperature of between 100 and 200°C for a period of between half an hour and one day.

20. Process according to any one of claims 17 to 19, **characterised in that** said compound having an electrophilic function is selected from among the halogenated or pseudohalogenated derivatives of organic sulphur compounds; the disulphides; the thiocyanates; and the carbonyl compounds.

21. Process according to claim 20, **characterised in that** said compound having an electrophilic function is a disulphide, notably an aryl disulphide.

22. Process according to claim 21, **characterised in that** said compound is a C₅₋₁₀ aryl disulphide optionally substituted by a group selected from among the C₁₋₁₀-alkyl, C₁₋₁₀-alkoxy, nitro and halo groups.

23. Process according to claim 19, **characterised in that** the carbonyl compound is selected from among the ketones, aldehydes, acid halides, activated esters and anhydrides.

24. Process according to claim 20, **characterised in that** the halogenated sulphur derivative is selected from among the sulphenyl, sulphinyl and sulphonyl halides.

25. Process according to any one of claims 17 to 20, **characterised in that** said compound having at least one electrophilic function does not comprise a hydrogen which can be removed by a strong base.

26. Use of a nucleophilic reagent comprising
a) a fluorocarboxylic acid of formula Ea-CF₂-COOH where Ea denotes an electron-attracting atom or group, at least partially salified by an organic or inorganic cation, the inorganic cation being selected from among sodium, potassium, rubidium, caesium and francium, and
b) a polar aprotic solvent;
wherein the content of releasable protons carried by its various components, including their impurities, is equal to not more than half the initial molar concentration of said fluorocarboxylic acid, and wherein the copper content is less than 1,000 ppm based on the initial content of said fluorocarboxylic acid, for grafting a substituted difluoromethyl group onto a compound comprising at least one electrophilic function, with the exception of the carbon atoms belonging to an aromatic nucleus.

27. Use according to claim 26, **characterised in that** said polar aprotic solvent is the compound having at least one electrophilic function.

28. Process according to any one of claims 17 to 25, **characterised in that** said compound comprising at least one electrophilic function acts as reagent and polar aprotic solvent.

## Patentansprüche

1. Nukleophiles Reagenz, **dadurch gekennzeichnet, daß** es
a) eine Fluorcarbonsäure der Formel Ea - CF₂ - COOH, worin Ea ein Elektronen ziehendes Atom oder eine Elektronen ziehende Gruppe bedeutet, die wenigstens teilweise mit einem organischen Kation oder Mineral ein Salz bildet und
b) ein aprotisches polares Lösungsmittel,
umfaßt, wobei der Gehalt an freisetzbaren Protonen dieser verschiedenen Komponenten einschließlich ihrer Verunreinigungen höchstens gleich der Hälfte der anfänglichen Molkonzentration der Fluorcarbonsäure ist und
wobei der Gehalt an Kupfer kleiner als 1000 ppm, bezogen auf den Anfangsgehalt der Fluorcarbonsäure ist.

2. Reagenz nach Anspruch 1 oder 2, **dadurch gekennzeichnet, daß** der Gehalt an Protonen höchstens gleich 10% der anfänglichen Molkonzentration des Fluorcarbonsäuresalzes beträgt.

3. Reagenz nach einem der vorangegangenen Ansprüche, **dadurch gekennzeichnet, daß** sein Wassergehalt unter 10% der Molkonzentration an der Fluorcarbonsäure beträgt.

4. Reagenz nach einem der vorangegangenen Ansprüche, **dadurch gekennzeichnet, daß** sein Gehalt an Übergangsmetallen, die mindestens in zwei stabilen Wertigkeiten vorliegen, kleiner als 1000 Mol ppm, bezogen auf das Fluorcarbonsäuresalz, betragen.

5. Reagenz nach einem der vorangegangenen Ansprüche, **dadurch gekennzeichnet, daß** der Gehalt der Elemente der Gruppe VIII des Periodensystems der Elemente kleiner als 100 Mol ppm, bezogen auf das Fluorcarbonsäuresalz, ist.

6. Reagenz nach einem der vorangegangenen Ansprüche, **dadurch gekennzeichnet, daß** sein Gehalt, ausgedrückt als Äquivalente an ionischem Fluorid höchstens gleich der anfänglichen Molkonzentration der Fluorcarbonsäure ist.

7. Reagenz nach einem der vorangegangenen Ansprüche, **dadurch gekennzeichnet, daß** der Donatorindex des polaren Lösungsmittels zwischen 10 und 30 beträgt.

8. Reagenz nach einem der vorangegangenen Ansprüche, **dadurch gekennzeichnet, daß** der Akzeptorindex des Lösungsmittels unter 20 liegt.

9. Reagenz nach einem der vorangegangenen Ansprüche, **dadurch gekennzeichnet, daß** der der ersten Azidität entsprechende pKa des Lösungsmittels wenigstens gleich 20 ist.

10. Reagenz nach einem der vorangegangenen Ansprüche, **dadurch gekennzeichnet, daß** es einen komplexierenden Kronenether enthält.

11. Reagenz nach einem der vorangegangenen Ansprüche, **dadurch gekennzeichnet, daß** das Elektronen ziehende Atom oder die Elektronen ziehende Gruppe ausgewählt ist aus den Elektronen ziehenden Gruppen, deren Hammetkonstante óₚ mindestens 0,1 ist.

12. Reagenz nach einem der vorangegangenen Ansprüche, **dadurch gekennzeichnet, daß** die Säure ausgewählt ist aus Verbindungen der Formel (1) X - CF₂ - COOH, worin X ein Halogenatom bedeutet, und den Verbindungen der Formel (2) R - G - CF₂ - COOH, worin R - G eine Nitrilgruppe bedeuten oder G C = 0, S = 0 oder -(CF₂)ₙ- mit n größer oder gleich 1 bedeuten und R ein organischer Rest oder ein indifferentes Mineral ist.

13. Reagenz nach einem der vorangegangenen Ansprüche, **dadurch gekennzeichnet, daß** die Säure oder das Fluorcarbonsäuresalz im Milieu des Reagenz vollständig löslich ist.

14. Reagenz nach einem der vorangegangenen Ansprüche, **dadurch gekennzeichnet, daß** das Salz ein Alkalimetallsalz, ausgewählt aus den Salzen von Natrium, Kalium, Rubidium, Cäsium, Francium, oder ein quaternäres Ammoniumsalz ist.

15. Reagenz nach einem der vorangegangenen Ansprüche, **dadurch gekennzeichnet, daß** die Lösungsmittel ausgewählt sind aus den disubstituierten N-Amiden, einschließlich der vierfach substituierten Harnstoffe und der monosubstituierten Lactame und der cyclischen und nicht-cyclischen Ether und dem Benzonitril.

16. Nukleophiles Reagenz nach Anspruch 1, **dadurch gekennzeichnet, daß** dieses Lösungsmittel wenigstens eine elektrophile Funktion aufweist.

17. Verfahren zur Herstellung einer Verbindung, die eine Difluormethylengruppe aufweist, **dadurch gekennzeichnet, daß** es
a) das Umsetzen eines Reagenz nach einem der Ansprüche 1 bis 16 mit einer Verbindung, die mindestens eine elektrophile Funktion aufweist, ausgenommen an einem aromatischen Kern auftretende elektrophile Kohlenstoffatome, und
b) das Erwärmen des erhaltenen Gemischs auf eine Temperatur zwischen 100 und 200°C über einen Zeitraum zwischen 0,5 Stunden und einem Tag umfaßt.

18. Verfahren nach Anspruch 17, **dadurch gekennzeichnet, daß** die wenigstens eine elektrophile Funktion tragende Verbindung ein aromatischer Aldehyd ist.

19. Verfahren zur Herstellung einer Verbindung, die eine Difluormethylengruppe aufweist, **gekennzeichnet durch** die Stufe des Erwärmens des Reagenz nach Anspruch 2 auf eine Temperatur zwischen 100 und 200°C über einen Zeitraum zwischen 0,5 Stunden und einem Tag.

20. Verfahren nach einem der Ansprüche 17 bis 19, **dadurch gekennzeichnet, daß** die elektrophile Funktion ausgewählt ist aus den halogenierten oder pseudohalogenierten Derivaten organischer Verbindungen des Schwefels, den Disulfiden, den Thiocyanaten und den carbonylierten Verbindungen.

21. Verfahren nach Anspruch 20, **dadurch gekennzeichnet, daß** die eine elektrophile Funktion aufweisende Verbindung ein Disulfid, insbesondere ein Aryldisulfid, ist.

22. Verfahren nach Anspruch 21, **dadurch gekennzeichnet, daß** die Verbindung ein C₅ bis C₁₀-Aryldisulfid, gegebenenfalls disubstituiert durch eine Gruppe, ausgewählt aus den C₁ bis C₁₀-Alkylgruppen, C₁ bis C₁₀-Alkoxygruppen, den Nitrogruppen und den Halogengruppen.

23. Verfahren nach Anspruch 19, **dadurch gekennzeichnet, daß** die carbonylierte Verbindung ausgewählt ist aus den Ketonen, Aldehyden, Säurehalogeniden, aktivierten Estern und Anhydriden.

24. Verfahren nach Anspruch 20, **dadurch gekennzeichnet, daß** das halogenierte Schwefelderivat ausgewählt ist aus den Sulfenylhalogenen, den Sulfinylhalogenen und den Sulfonylhalogenen.

25. Verfahren nach einem der Ansprüche 17 bis 20, **dadurch gekennzeichnet, daß** die Verbindung, die mindestens eine elektrophile Funktion aufweist, keinen Wasserstoff trägt, der durch eine starke Base entziehbar ist.

26. Verwendung eines nukleophilen Reagenz, umfassend
a) eine Fluorcarbonsäure der Formel Ea - CF₂ - COOH, worin Ea ein Elektronen ziehendes Atom oder eine Elektronen ziehende Gruppe bedeutet, die wenigstens teilweise mit einem organischen Kation oder Mineral ein Salz bildet und
b) ein aprotisches polares Lösungsmittel,
wobei der Gehalt an freisetzbaren Protonen dieser verschiedenen Komponenten einschließlich ihrer Verunreinigungen höchstens gleich der Hälfte der anfänglichen Molkonzentration der Fluorcarbonsäure ist und
wobei der Gehalt an Kupfer kleiner als 1000 ppm, bezogen auf den Anfangsgehalt der Fluorcarbonsäure, ist, zum Pfropfen einer Difluormethylgruppe, die durch eine Verbindung substituiert ist, die wenigstens eine elektrophile Funktion aufweist, ausgenommen an einem aromatischen Kern auftretende Kohlenstoffatome.

27. Verwendung nach Anspruch 26, **dadurch gekennzeichnet, daß** das polare aprotische Lösungsmittel eine Verbindung ist, die mindestens eine elektrophile Funktion aufweist.

28. Verfahren nach einem der Ansprüche 17 bis 25, **dadurch gekennzeichnet, daß** die Verbindung, die mindestens eine elektrophile Funktion aufweist, die Rolle des Reagenz und des polaren aprotischen Lösungsmittels spielt.
